(19) Europäisches Patentamt
European Patent Office
Office européen des brevets

(11) Veröffentlichungsnummer : **0 075 242**
**B1**

(12) **EUROPÄISCHE PATENTSCHRIFT**

(45) Veröffentlichungstag der Patentschrift : **13.03.85**

(21) Anmeldenummer : **82108428.2**

(22) Anmeldetag : **13.09.82**

(51) Int. Cl.⁴ : **C 07 C 91/06**, C 07 C 93/04, C 07 C 87/62, C 07 C143/75, A 61 K 7/13

(54) **N-Substituierte 2.4-Diamino-m-Xylole und diese enthaltende Haarfärbemittel.**

(30) Priorität : **21.09.81 DE 3137473**

(43) Veröffentlichungstag der Anmeldung : **30.03.83 Patentblatt 83/13**

(45) Bekanntmachung des Hinweises auf die Patenterteilung : **13.03.85 Patentblatt 85/11**

(84) Benannte Vertragsstaaten : **AT BE CH DE FR GB IT LI LU NL SE**

(56) Entgegenhaltungen : **US-A- 3 963 764**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Konrad, Günther, Dr.**
**Feuerbach-Weg 12**
**D-4010 Hilden (DE)**
Erfinder : **Maak, Norbert, Dr.**
**Liebigstrasse 18**
**D-4040 Neuss (DE)**

**EP 0 075 242 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue, N-substituierte 2.4-Diamino-m-Xylole sowie Mittel zur oxidativen Färbung von Haaren, welche diese N-substituierten 2.4-Diamino-m-Xylole als Kupplungskomponente enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden Phenole, Naphthole, Resorcinderivate und Pyrazolone genannt.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein. Weiterhin ist von Bedeutung, daß auf dem zu färbenden Haar möglichst kräftige und den natürlichen Haarfarbnuancen weitgehend entsprechende Farbtöne erhalten werden. Ferner kommt der allgemeinen Stabilität der gebildeten Farbstoffe sowie deren Lichtechtheit, Waschechtheit und Thermostabilität ganz besondere Bedeutungh zu, um Farbverschiebungen von der ursprünglichen Farbnuance oder gar Farbumschläge in andere Farbtöne zu vermeiden.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben gelangt, welche den gestellten Anforderungen in besonders hohem Maße gerecht werden, wenn man als Kupplerkomponenten die nachstehend beschriebenen neuen Verbindungen in Kombination mit üblichen Entwicklersubstanzen verwendet.

Gegenstand der Erfindung sind 2.4-Diamino-m-Xylole der allgemeinen Formel I

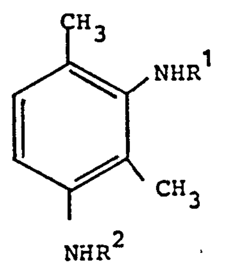

(I)

in welcher jeweils einer der beiden Reste, $R^1$ oder $R^2$, Wasserstoff ist, der andere eine Alkylgruppe mit 1-4 Kohlenstoffatomen, eine Alkylsulfonylgruppe mit 1-4 Kohlenstoffatomen, eine Mono- oder Dihydroxyalkylgruppe mit 2-4 Kohlenstoffatomen, eine Alkoxyalkylgruppe mit 1-4 Kohlenstoffatomen im Alkoxyrest und 2-4 Kohlenstoffatomen im Alkylrest, eine Aminoalkylgruppe mit 2-4 Kohlenstoffatomen, die gegebenenfalls am Stickstoffatom durch eine oder zwei Alkylgruppen mit 1-4 Kohlenstoffatomen oder durch eine oder zwei Hydroxyalkylgruppen mit 2-4 Kohlenstoffatomen substituiert ist, oder eine 2-Hydroxyethoxyethylgruppe darstellt.

Ein weiterer Gegenstand der Erfindung sind Haarfärbemittel, bestehend aus mindestens einem N-substituierten 2.4-Diamino-m-Xylol der Formel I oder dessen anorganischem oder organischem Salz, mindestens einer üblichen Entwicklungskomponente, gegebenenfalls weiteren üblichen Kupplerkomponenten sowie gegebenenfalls üblichen direktziehenden Farbstoffen und üblichen weiteren Zusätzen.

Bei ihrem Einsatz als Kupplerkomponenten liefern die erfindungsgemäßen Verbindungen der Formel I mit den im allgemeinen für die Oxidationshaarfärbung verwendeten Entwicklersubstanzen sehr intensive Farbtöne, die, je nach Art der verwendeten Entwicklersubstanz die Erzeugung von grüngrauen, rotbraunen, schwarzblauen oder dunkelvioletten Farbtönen ermöglichen. Die neuen Kupplersubstanzen stellen somit eine wesentliche Bereicherung der oxidativen Haarfärbemöglichkeiten dar. Die erfindungsgemäßen 2.4-Diamino-m-Xylole sind außerdem gut wasserlöslich und daher leicht in Haarfärbemitteln zu verarbeiten. Sie zeigen eine sehr niedrige akute Giftigkeit und sind daher als toxikologisch und dermatologisch unbedenklich anzusehen. Darüber hinaus zeichnen sich die mit den erfindungsgemäßen 2.4-Diamino-m-Xylolen hergestellten Oxidationshaarfarben durch sehr gute Echtheitseigenschaften der damit erzielten Färbungen aus.

Oxidationshaarfärbemittel mit einem Gehalt an den erfindungsgemäßen 2.4-Diamino-m-Xylolender Formel I und/oder deren anorganischen oder organischen Salzen stellen daher besonders wertvolle Kompositionen auf dem Gebiet der Oxidationshaarfärbemittel dar.

Die Herstellung der erfindungsgemäßen N-substituierten 2.4-Diamino-m-Xylole der Formel I erfolgt zweckmäßig ausgehend vom unsubstituierten 2.4-Diamino-m-Xylol. Diese Verbindung ist durch katalytische Hydrierung aus dem im Handel erhältlichen 2.4-Dinitro-m-Xylol zugänglich. Die katalytische Hydrierung erfolgt durch Umsetzung mit Wasserstoff in Gegenwart von Hydrierkatalysatoren wie z. B. Palladium auf Aktivkohle nach an sich bekanntem Verfahren. Zur Herstellung der erfindungsgemäßen N-substituierten 2.4-Diamino-m-Xylole wird 2.4-Diamino-m-Xylol im Molverhältnis 1 : 1 mit geeigneten Alkylierungsmitteln in Gegenwart von Basen umgesetzt. Als geeignete Alkylierungsmittel kommen Verbindungen der allgemeinen Formel R-X infrage, in welchen X ein Halogenatom darstellt und R eine Alkylgruppe mit 1-4 Kohlenstoffatomen, eine Alkylsulfonylgruppe mit 1-4 Kohlenstoffatomen, eine Mono- oder Dihydroxyalkylgruppe mit 2-4 Kohlenstoffatomen, eine Alkoxyalkylgruppe mit 1-4 Kohlenstoffatomen im Alkoxyrest und 2-4 Kohlenstoffatomen im Alkylrest oder eine Aminoalkylgruppe mit 2-4 Kohlenstoffatomen darstellt, die gegebenenfalls am Stickstoffatom durch eine oder zwei Alkylgruppen mit 1-4 Kohlenstoffatomen oder durch eine oder zwei Hydroxyalkylgruppen mit 2-4 Kohlenstoffatomen substituiert ist. Geeignete Alkylierungsmittel sind z. B. 2-Bromethanol, 2-Bromethyl-ethylether, 2-Diethylaminoethylchlorid, 2-(2-Chlorethoxy)-ethanol, Methansulfonsäurechlorid usw. Geeignete Basen sind z. B. Triethylamin oder Calciumcarbonat. Die Alkylierung kann auch mit geeigneten Oxiranverbindungen durchgeführt werden, z. B. mit Alkylenoxiden mit 1-4 Kohlenstoffatomen oder Glycidol.

Bei der Alkylierung des 2.4-Diamino-m-xylols kann die Substitution sowohl an der Aminogruppe in 2-Position als auch an der Aminogruppe in 4-Position stattfinden. Obwohl die Alkylierung der Aminogruppen in 4-Position leicht bevorzugt ist, entstehen stets Mischungen der beiden strukturisomeren Verbindungen. Die beiden Verbindungen lassen sich zwar durch an sich bekannte Methoden voneinander trennen und rein darstellen, z. B. durch Säulenchromatographie oder durch präparative Dünnschichtchromatographie. Für die Verwendung als Kupplerkomponente in Oxidationshaarfärbemitteln ist jedoch das Gemisch der Isomeren, an der Aminogruppe in 2-Position und der an der Aminogruppe in 4-Position substituierten 2.4-Diamino-m-xylole in gleicher Weise geeignet und zeigt alle die genannten anwendungstechnischen, toxikologischen und färbetechnischen Vorzüge.

Beispiele für erfindungsgemäße 2.4-Diamino-m-xylole sind z. B. 2(4)-Amino-4(2)-(2-hydroxyethyl)-amino-m-xylol, 2(4)-Amino-4(2)-[(2-hydroxypropyl)-amino]-m-xylol, 2(4)-Amino-4(2)-[(2-ethoxyethyl)-amino]-m-xylol, 2(4)-Amino-4(2)-[(2.3-dihydroxypropyl)-amino]-m-xylol, 2(4)-Amino-4(2)-[(2-diethylaminoethyl)-amino]-m-xylol, 2(4)-Amino-4(2)-[2-di-(2-hydroxyethyl)-aminoethyl]-amino-m-xylol 2(4)-Amino-4(2)-[2-(2-hydroxy-ethoxy)-ethyl]-amino-m-xylol.

Die erfindungsgemäß als Kupplerkomponenten zu verwendenden substituierten 2.4-Diamino-m-xylole können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Als Beispiele für Entwicklersubstanzen, die in den erfindungsgemäßen Haarfärbemitteln eingesetzt werden können, sind primäre aromatische Amine mit einer weiteren in p-Stellung befindlichen funktionellen Gruppe wie p-Phenylendiamin, p-Toluylendiamin, p-Amino-phenol, N-Methyl-p-phenylendiamin, N,N-Dimethyl-p-phenylkendiamin, N,N-Diethyl-2-methyl-p-phenylendiamin, N-Ethyl-N-hydroxyethyl-p-phenylendiamin, Chlor-p-phenylendiamin, N,N-Bis-hydroxy-ethylamino-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR'-Gruppen, $NR_2$'-Gruppen, wobei R' einen Alkyl- oder Hydroxyalkylrest mit 1-4 Kohlenstoffatomen darstellt, ferner Diaminopyridin-derivate, heterocyclische Hydrazonderivate wie 1-Methyl-pyrrolidon-(2)-hydrazon, 4-Aminopyrazolonderivate wie 4-Amino-1-phenyl-3-carbamoyl-pyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methyl-aminopyrimidin, 2,4,5-Triamino-6-dimethylaminopyrimidin, 2,4,5-Triamino-6-piperidinopyrimidin, 2,4,5-Triamino-6-anilino-pyrimidin, 2,4,5-Triamino-6-morpholinopyrimidin, 2,4,5-Triamino-6-β-hydroxy-ethylamino-pyrimidin anzuführen.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß zu verwendenden Entwicklerverbindungen als auch die Kupplersubstanz Gemische der erfindungsgemäß einzusetzenden N-substituierten 2,4-Diamino-m-xylole darstellen.

Es ist insbesondere aus wirtschaftlichen Gründen vorteilhaft, die bei der beschriebenen Synthese der erfindungsgemäßen 2,4-Diamino-m-xylole anfallenden Gemische der in 2-Position und der in 4-Position substituierten 2,4-Diamino-m-xylole direkt ohne Isolierung der einzelnen Isomeren einzusetzen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich wie bei anderen Oxidationshaarfarbstoffen auch, durch Luftsauerstoff erfolgen. Zweckmäßigerweise werden jedoch

chemische Oxidationsmittel eingesetzt. Als soche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxidisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z. B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie z. B. Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die nachfolgenden Beispiele sollen den Erfindungsgegenstand näher erläutern, ohne ihn jedoch hierauf zu beschränken.

## Beispiele

Zunächst wird die Herstellung der in den folgenden Beispielen für die verschiedenen Ausfärbungen als Kupplerkomponenten eingesetzten erfindungsgemäßen N-substituierten 2.4-Diamino-m-xylole (K1-6) beschrieben.

1. 2(4)-Amino-4(2)-[(2-hydroxyethyl)-amino]-m-xylol (K1)

4,1 g 2,4-Diamino-m-xylol (0,03 Mol), 4,1 g 2-Bromethanol (0,0328 Mol) und 2 g Calciumcarbonat wurden in 125 ml Wasser gegeben und unter Rühren 4 Stunden unter Rückflußkühlung zum Sieden erhitzt. Nach Abkühlung wurde vom ungelösten Festkörper abfiltriert. Das eingeengte Filtrat wurde mit Diethylether extrahiert. Nach Einengen des Ether-Extraktes wurden 4,1 g eines dunklen Öles erhalten.

Elementaranalyse für $C_{10}H_{16}N_2O$

| | | | | | |
|---|---|---|---|---|---|
| berechnet | (Gew.-%) | C 66,64 | H 8,95 | N 15,54 | O 8,88 |
| gefunden | (Gew.-%) | C 66,4 | H 8,79 | N 15,6 | O 8,82 |

2. 2(4)-Amino-4(2)-[(2-ethoxyethyl)-amino]-m-xylol (K2)

4,1 g 2,4-Diamino-m-xylol (0,03 Mol), 4,6 g 2-Bromethyl-ethylether (0,03 Mol) und 2 g Calciumcarbonat wurden in 125 ml Wasser gegeben und unter Rühren 4 Stunden unter Rückflußkühlung zum Sieden erhitzt. Nach Abkühlung wurde vom ungelösten Fettkörper abfiltriert. Das eingeengte Filtrat wurde mit Diethylether extrahiert. Nach Einengen des Ether-Extraktes wurden 3,7 g eines viskosen Öles erhalten.

Elementaranalyse für $C_{12}H_{20}N_2O$

| | | | | |
|---|---|---|---|---|
| berechnet | (Gew.-%) | C 69,19 | H 9,68 | N 13,45 |
| gefunden | (Gew.-%) | C 69,5 | H 9,91 | N 13,4 |

3. 2(4)-Amino-4(2)-[(2.3-dihydroxypropyl)-amino]-m-xylol (K3)

13,6 g 2.4-Diamino-m-xylol (0,1 Mol), 8,7 g Glycidol (85 %ig) (0,1 Mol) und 0,05 g Tetramethylammoniumchlorid wurden in 100 ml Methanol unter Rühren 6 Stunden auf 60 °C erhitzt. Nach dem Abkühlen wurde die Reaktionslösung eingedampft. Als Rückstand wurden 20 g eines dunklen öligen Produktes erhalten. Die Identifikation durch Massenspektrum ergab ein Molekülion von 210 Einheiten.

Daneben wurden geringe Mengen des Anlagerungsproduktes von Glyceringlycidylether an 2.4-Diamino-m-xylol im Massenspektrum als Molekülion mit 284 Einheiten idenfiziert.

4. 2(4)-Amino-4(2)-[(2-diethylaminoethyl)-amino]-m-xylol (K4)

4,1 g 2.4-Diamino-m-xylol (0,03 Mol), 7 g Calciumcarbonat und 80 ml Wasser wurden unter Rühren zum Sieden erhitzt. Dann wurden 5,2 g Diethylamino-ethylchlorid-hydrochlorid (0,03 Mol) portionsweise zugesetzt. Das Reaktionsgemisch wurde nach 10 Stunden unter Rückflußkühlung zum Sieden erhitzt. Nach dem Abkühlen wurde vom ungelösten Festkörper abfiltriert. Das Filtrat wurde durch Zusatz von verdünnter Natronlauge auf PH9 eingestellt, eingeengt und mit Diethylether extrahiert. Nach Eingengen des Ether-Extraktes wurden 5,9 g eines viskosen Öles erhalten.

Elementaranalyse : $C_{14}H_{25}N_3$

| | | | | |
|---|---|---|---|---|
| berechnet | (Gew.-%) | C 71,44 | H 10,71 | N 17,85 |
| gefunden | (Gew.-%) | C 71,30 | H 11,20 | N 18,00 |

5. 2(4)-Amino-4(2)-[2-(2-hydroxy-ethoxy)-ethyl]-amino-m-xylol (K5)

6,8 g 2,4-Diamino-m-xylol (0,05 Mol), 6,3 g 2-(2-Chlor-ethoxy)-ethanol (0,063 Mol) und 3 g Calciumcarbonat wurden in 125 ml Wasser gegeben und unter Rühren und Rückflußkühlung 8 Stunden zum Sieden erhitzt. Nach dem Abkühlen und 12 stündigem Stehen wurde vom Bodenkörper abfiltriert. Das Filtrat wurde eingeengt, durch Zugabe von verdünnter Natronlauge alkalisch eingestellt und mit Diethylether extrahiert. Der Ether-Extrakt wurde eingeengt und ergab als Rückstand 6,5 g eines viskosen Öles.

Elementaranalyse : $C_{12}H_{20}N_2O_2$

| | | | | |
|---|---|---|---|---|
| berechnet | (Gew.-%) | C 64,26 | H 8,99 | N 12,49 |
| gefunden | (Gew.-%) | C 64,50 | H 8,89 | N 12,20 |

6. 2(4)-Amino-4(2)-methansulfonylamino-m-xylol (K6)

6,8 g 2,4-Diamino-m-xylol (0,05 Mol) und 5,1 g Triethylamin wurden in 100 ml Toluol gelöst. In dieser Lösung wurden 5,7 g Methansulfonsäurechlorid (0,05 Mol) zugetropft. Nach beendeter Zugabe wurde noch 2 Stunden bei 20 °C gerührt. Das ausgeschiedene Produkt wurde abfiltriert und mit wenig Wasser gewaschen. Das Produkt wurde zunächst aus Wasser, dann aus Toluol umkristallisiert. Dabei wurden 8,1 g Reaktionsprodukt mit einem Schmelzpunkt von 123 °C gewonnen.

Elementaranalyse : $C_9H_{14}N_2O_2S$

| | | | | |
|---|---|---|---|---|
| berechnet | (Gew.-%) | C 50,69 | H 13,13 | S 15,03 |
| gefunden | (Gew.-%) | C 50,3 | H 13,0 | S 15,0 |

Die erfindungsgemäßen Kupplerkomponenten K1-K6 wurden mit den folgenden Entwicklerkomponenten in Haarfärbemittel eingesetzt :

E1 : 2.4.5.6-Tetraaminopyrimidin-sulfat
E2 : p-Toluylendiamin-sulfat
E3 : N-Methyl-p-phenylendiamin-sulfat
E4 : p-Phenylendiamin-sulfat
E5 : p-Aminophenol
E6 : 2,5-Diaminoanisol-sulfat

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$,
10 Gewichtsteilen Fettalkoholsulfat (Natriumsalz) der Kettenlänge $C_{12}$-$C_{18}$,
75 Gewichtsteilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit zusätzlichem Oxidationsmittel wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | Kuppler | Entwickler | erhaltene Anfärbung mit 1 %iger $H_2O_2$ - Lösung |
|---|---|---|---|
| 1 | K 1 | E 1 | grüngrau |
| 2 | K 2 | E 1 | olivbraun |
| 3 | K 3 | E 1 | braun |
| 4 | K 4 | E 1 | hellbraun |
| 5 | K 5 | E 1 | oliv |
| 6 | K 6 | E 1 | mattgrün |
| 7 | K 1 | E 2 | dunkelviolett |
| 8 | K 2 | E 2 | schwarzblau |
| 9 | K 3 | E 2 | dunkelviolett |
| 10 | K 4 | E 2 | dunkelrubin |
| 11 | K 5 | E 2 | schwarzblau |
| 12 | K 6 | E 2 | braungrau |
| 13 | K 1 | E 3 | dunkelviolett |
| 14 | K 2 | E 4 | schwarzviolett |
| 15 | K 2 | E 5 | dunkelmagenta |
| 16 | K 5 | E 6 | dunkelblau |
| 17 | K 5 | E 3 | blauschwarz |
| 18 | K 5 | E 4 | blauschwarz |
| 19 | K 6 | E 4 | dunkelbraun |

**Ansprüche** (für die Vertragsstaaten : BE, CH, DE, FR, GB, IT, LI, LU, NL, SE)

1. 2.4-Diamino-m-xylole der allgemeinen Formel I

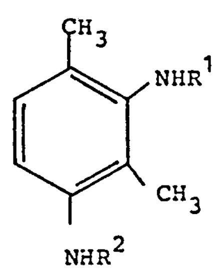

(I)

in welcher jeweils einer der beiden Reste $R^1$ oder $R^2$ Wasserstoff ist, der andere eine Alkylgruppe mit 1-4 Kohlenstoffatomen, eine Alkylsulfonylgruppe mit 1-4 Kohlenstoffatomen, eine Mono- oder Dihydroxyalkylgruppe mit 2-4 Kohlenstoffatomen, eine Alkoxyalkylgruppe mit 1-4 Kohlenstoffatomen im Alkoxyrest und 2-4 Kohlenstoffatomen im Alkylrest, eine Aminoalkylgruppe mit 2-4 Kohlenstoffatomen, die gegebenenfalls am Stickstoffatom durch eine oder zwei Alkylgruppen mit 1-4 Kohlenstoffatomen oder durch eine oder zwei Hydroxyalkylgruppen mit 2-4 Kohlenstoffatomen substituiert ist, oder eine 2-Hydroxyethoxyethylgruppe darstellt.

2. Haarfärbemittel, bestehend aus mindestens einem N-substituierten 2.4-Diamino-m-xylol oder dessen anorganischem oder organischem Salz, mindestens einer üblichen Entwicklungskomponente, gegebenenfalls weiteren üblichen Kupplungskomponenten sowie gegebenenfalls üblichen direktziehenden Farbstoffen und üblichen weiteren Zusätzen, dadurch gekennzeichnet, daß die N-substituierten 2.4-Diamino-m-xylole Verbindungen der allgemeinen Formel I nach Anspruch 1 sind.

3. Haarfärbemittel nach Anspruch 2, gekennzeichnet durch einen Gehalt an Entwickler-Kuppler-Kombination von 0,2 bis 5 Gew.-%, vorzugsweise 1 bis 3 Gew.-% bezogen auf das gesamte Mittel.

**Ansprüche** (für den Vertragsstaat AT)

1. Verfahren zur Herstellung von 2.4-Diamino-m-xylolen der allgemeinen Formel I

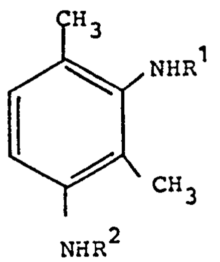

(I)

in welcher jeweils einer der beiden Reste $R^1$ oder $R^2$ Wasserstoff ist, der andere eine Alkylgruppe mit 1-4 Kohlenstoffatomen, eine Alkylsulfonylgruppe mit 1-4 Kohlenstoffatomen, eine Mono- oder Dihydroxyalkylgruppe mit 2-4 Kohlenstoffatomen, eine Alkoxyalkylgruppe mit 1-4 Kohlenstoffatomen im Alkoxyrest und 2-4 Kohlenstoffatomen im Alkylrest oder eine Aminoalkylgruppe mit 2-4 Kohlenstoffatomen, die gegebenenfalls am Stickstoffatom durch eine oder zwei Alkylgruppen mit 1-4 Kohlenstoffatomen oder durch eine oder zwei Hydroxyalkylgruppen mit 2-4 Kohlenstoffatomen substituiert ist oder eine 2-Hydroxyethoxyethylgruppe darstellt, durch katalytische Hydrierung von 2.4-Dinitro-m-xylol und Umsetzung des dabei entstehenden 2.4-Diamino-m-xylols mit 1 Mol eines Alkylierungsmittels der allgemeinen Formel R—X, worin X ein Halogenatom ist und R die für $R^1$ und $R^2$ genannte Bedeutung mit Ausnahme von Wasserstoff hat.

2. Haarfärbemittel, bestehend aus mindestens einem N-substituierten 2.4-Diamino-m-xylol oder dessen anorganischem oder organischem Salz, mindestens einer üblichen Entwicklungskomponente, gegebenenfalls weiteren üblichen Kupplungskomponenten sowie gegebenenfalls üblichen direktziehenden Farbstoffen und üblichen weiteren Zusätzen, dadurch gekennzeichnet, daß die N-substituierten 2.4-Diamino-m-xylole Verbindungen der allgemeinen Formel I nach Anspruch 1 sind.

3. Haarfärbemittel nach Anspruch 2, gekennzeichnet durch einen Gehalt an Entwickler-Kuppler-Kombination von 0,2 bis 5 Gew.-%, vorzugsweise 1 bis 3 Gew.-%, bezogen auf das gesamte Mittel.

**Claims** (for the Contracting States : BE, CH, DE, FR, GB, IT, LI, LU, NL, SE)

1. 2,4-Diamino-m-xylenes corresponding to the following general formula

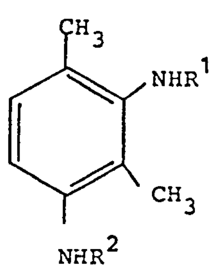

(I)

in which one of the two radicals $R^1$ or $R^2$ is hydrogen and the other is an alkyl group containing from 1 to 4 C-atoms, an alkyl sulfonyl group containing from 1 to 4 C-atoms, a mono- or dihydroxyalkyl group containing from 2 to 4 C-atoms, an alkoxyalkyl group containing from 1 to 4 C-atoms in the alkoxy radical and from 2 to 4 C-atoms in the alkyl radical, an aminoalkyl group containing from 2 to 4 C-atoms optionally substituted at the nitrogen atom by one or two $C_1$-$C_4$ alkyl groups or by one or two $C_2$-$C_4$ hydroxyalkyl groups or a 2-hydroxyethoxyethyl group.

2. Hair dyes consisting of a least one N-substituted 2,4-diamino-m-xylene or an inorganic or organic salt thereof, at least one standard developing component, optionally other standard coupling components and, optionally, standard substantive dyes and other standard additives, characterized in that the N-substituted 2,4-diamino-m-xylenes are compounds corresponding to general formula I in Claim 1.

3. Hair dyes as claimed in Claim 2, characterized by a content of from 0.2 to 5 % by weight and preferably from 1 to 3 % by weight, based on the hair dye as a whole, of developer-coupler combination.

**Claims** (for the Contracting State AT)

1. A process for the production of 2,4-diamino-m-xylenes corresponding to the following general formula

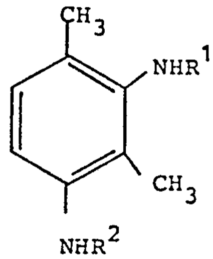

(I)

in which one of the two radicals $R^1$ or $R^2$ is hydrogen and the other is an alkyl group containing from 1 to 4 C-atoms, an alkyl sulfonyl group containing from 1 to 4 C-atoms, a mono- or dihydroxyalkyl group containing from 2 to 4 C-atoms, an alkoxyalkyl group containing from 1 to 4 C-atoms in the alkoxy radical and from 2 to 4 C-atoms in the alkyl radical or an aminoalkyl group containing from 2 to 4 C-atoms optionally substituted at the nitrogen atom by one or two $C_1$-$C_4$ alkyl groups or by one or two $C_2$-$C_4$ hydroxyalkyl groups or a 2-hydroxyethoxyethyl group, by catalytically hydrogenating 2,4-dinitro-m-xylene and reacting the 2,4-diamino-m-xylene formed with 1 mole of an alkylating agent corresponding to the general formula R—X, in which X is a halogen atom and R has the same meaning as $R^1$ and $R^2$ except for hydrogen.

2. Hair dyes consisting of a least one N-substituted 2,4-diamino-m-xylene or an inorganic or organic salt thereof, at least one standard developing component, optionally other standard coupling components and, optionally, standard substantive dyes and other standard additives, characterized in that the N-substituted 2,4-diamino-m-xylenes are compounds corresponding to general formula I in Claim 1.

3. Hair dyes as claimed in Claim 2, characterized by a content of from 0.2 to 5 % by weight and preferably from 1 to 3 % by weight, based on the hair dye as a whole, of developer-coupler combination.

**Revendications** (pour les Etats contractants : BE, CH, DE, FR, GB, IT, LI, LU, NL, SE)

1. 2,4-diamino-m-xylènes de formule générale I

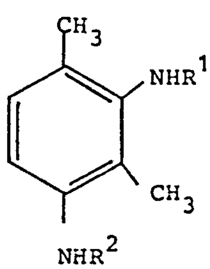

(I)

dans laquelle un des deux restes $R^1$ ou $R^2$ est de l'hydrogène, l'autre, un groupe alcoyle avec 1 à 4 atomes de carbone, un groupe alcoyl-sulfonyle avec 1 à 4 atomes de carbone, un groupe mono- ou dihydroxyalcoyle avec 2 à 4 atomes de carbone, un groupe alcoxyalcoyle avec 1 à 4 atomes de carbone dans le reste alcoxy et 2 à 4 atomes de carbone dans le reste alcoyle, un groupe aminoalcoyle avec 2 à 4 atomes de carbone, éventuellement substitué sur l'azote par un ou deux groupes alcoyle avec 1 à 4 atomes de carbone ou par un ou deux groupes hydroxyalcoyle avec 2 à 4 atomes de carbone, ou, constitué par un groupe 2-hydroxy-éthoxyéthyle.

2. Colorants pour cheveux, constitués par au moins un 2,4-diamino-m-xylène N-substitué ou ses sels minéraux ou organiques, au moins un composant habituel de développement, éventuellement d'autres

composants habituels de copulation, ainsi qu'éventuellement des colorants directs courants, et d'autres additifs courants, colorants caractérisés en ce que les 2,4-diamino-m-xylènes N-substitués sont des composés selon la formule I de la revendication 1.

3. Colorants pour cheveux selon la revendication 2, caractérisé par une teneur en combinaison produit de développement-produit de copulation de 0,2 à 5 % en poids, de préférence de 1 à 3 % en poids, calculé sur la totalité de l'agent colorant.

**Revendications** (pour l'Etat contractant AT)

1. Procédé de préparation de 2,4-diamino-m-xylènes de formule générale I

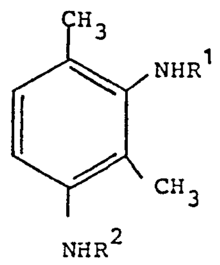

(I)

dans laquelle un des deux restes $R^1$ ou $R^2$ est de l'hydrogène, l'autre un groupe alcoyle avec 1 à 4 atomes de carbone, un groupe alcoylsulfonyl avec 1 à 4 atomes de carbone, un groupe mono- ou dihydroxyalcoyle avec 2 à 4 atomes de carbone, un groupe alcoxy-alcoyle avec 1 à 4 atomes de carbone dans le reste alcoxy et 2 à 4 atomes de carbone dans le reste alcoyle, ou un groupe amino-alcoyle avec 2 à 4 atomes de carbone, éventuellement substitué sur l'atome d'azote par un ou deux groupes alcoyle avec 1 à 4 atomes de carbone ou par un ou deux groupes hydroxyalcoyle avec 2 à 4 atomes de carbone, ou constitué par un groupe 2-hydroxy-éthoxy-éthyle, par hydrogénation catalytique de 2,4-dinitro-m-xylènes et réaction des 2,4-diamino-m-xylènes obtenus avec 1 mole d'un agent d'alcoylation de formule générale R-X, où X est un atome d'halogène et R a la signification mentionnée pour $R^1$ et $R^2$, à l'exception de l'hydrogène.

2. Colorants pour cheveux, constitués par au moins un 2,4-diamino-m-xylène N-substitué ou ses sels minéraux ou organiques, au moins un composant de développement courant, éventuellement d'autres composants de copulation habituels ainsi qu'éventuellement des colorants directs courants, et d'autres additifs usuels, colorants caractérisés en ce que les 2,4-m-xylènes sont des composés de formule générale I selon la revendication 1.

3. Colorants pour cheveux selon la revendication 2, caractérisés par une teneur en combinaison produit de développement-produit de copulation de 0,2 à 5 % en poids, de préférence 1 à 3 % en poids, calculé sur la totalité de l'agent colorant.